# EUROPEAN PATENT SPECIFICATION

(11) **EP 3 619 714 B1**
(45) Date of publication and mention of the grant of the patent: **12.08.2026**
(21) Application number: 18720579.4
(22) Date of filing: 25.04.2018
(51) Int. Cl.: G16H 30/20

(54) **DYNAMIC SYSTEM FOR DELIVERING FINDING-BASED RELEVANT CLINICAL CONTEXT IN IMAGE INTERPRETATION ENVIRONMENT**
DYNAMISCHES SYSTEM ZUR ERMITTLUNG EINES BEFUNDBASIERTEN RELEVANTEN KLINISCHEN KONTEXTES IN EINER BILDINTERPRETATIONSUMGEBUNG
SYSTÈME DYNAMIQUE POUR FOURNIR UN CONTEXTE CLINIQUE PERTINENT BASÉ SUR LA RECHERCHE DANS UN ENVIRONNEMENT D'INTERPRÉTATION D'IMAGES.

(30) Priority: 05.05.2017 US 201762501853 P
(43) Date of publication of application: 11.03.2020
(73) Proprietor: Koninklijke Philips N.V., 5656 AE Eindhoven (NL)
(72) Inventor: SEVENSTER, Merlijn, 5656 AE Eindhoven (NL); TAHMASEBI MARAGHOOSH, Amir, Mohammad, 5656 AE Eindhoven (NL); SPENCER, Kirk, 5656 AE Eindhoven (NL)
(74) Representative: Philips Intellectual Property & Standards
(86) International application number: PCT/EP2018/060513
(87) International publication number: WO 2018/202482

(56) References cited:
- WO-A1-2014/115065
- US-A1- 2012 131 436
- US-A1- 2015 149 215
- DAVID S CHANNIN ET AL: "The caBIG(TM) Annotation and Image Markup Project", JOURNAL OF DIGITAL IMAGING ; THE JOURNAL OF THE SOCIETY FOR COMPUTER APPLICATIONS IN RADIOLOGY, SPRINGER-VERLAG, NE, vol. 23, no. 2, 18 March 2009 (2009-03-18), pages 217 - 225, XP019785188, ISSN: 1618-727X

## Description

### FIELD

The following relates generally to the image interpretation workstation arts, radiology arts, echocardiography arts, and related arts.

### BACKGROUND

An image interpretation workstation provides a medical professional such as a radiologist or cardiologists with the tools to view images, manipulate images by operations such as pan, zoom, three-dimensional (3D) rendering or projection, and so forth, and also provides the user interface for selecting and annotating portions of the images and for generating an image examination findings report. As an example, in a radiology examination workflow, a radiology examination is ordered and the requested images are acquired using a suitable imaging device, e.g. a magnetic resonance imaging (MRI) device for MR imaging, a positron emission tomography (PET) imaging device for PET imaging, a gamma camera for single photon emission computed tomography (SPECT) imaging, a transmission computed tomography (CT) imaging device for CT imaging, or so forth. The medical images are typically stored in a Picture Archiving and Communication System (PACS), or in a specialized system such as a cardiovascular information system (CVIS). After the actual imaging examination, a radiologist operating a radiology interpretation workstation retrieves the images from the PACS, reviews them on the display of the workstation, and types, dictates, or otherwise generates a radiology findings report.

As another illustrative workflow example, an echocardiogram is ordered, and an ultrasound technician or other medical professional acquires the requested echocardiogram images. A cardiologist or other professional operating an image interpretation workstation retrieves the echocardiogram images, reviews them on the display of the workstation, and types, dictates, or otherwise generates an echocardiogram findings report.

In such imaging examinations, the radiologist, cardiologist, or other medical professional performing the image interpretation can benefit from reviewing the patient's medical record (i.e. patient record), which may contain information about the patient that is informative in drawing appropriate clinical findings from the images. The patient's medical record is preferably stored electronically in an electronic database such as an electronic medical record (EMR), an electronic health record (EHR), or in a domain-specific electronic database such as the aforementioned CVIS for cardiovascular treatment facilities. To this end, it is known to provide access to the patient record stored at the EMR, EHR, CVIS, or other database via the image interpretation workstation. For example, the image interpretation environment may execute as one program running on the workstation, and the EMR interface may execute as a second program running concurrently on the workstation.

WO 2014/115065 A1 relates to an apparatus for processing of medical images for receiving a an image representing characteristics of a part of a human or animal body. A matching unit determines a set of matching samples from a set of samples in response to a comparison of an image associated set of signatures to a sample associated sets of signatures of the set of samples. A decision unit then determines medical data for the image in response to the medical data comprised in the samples of the set of matching sample.

Document D2 (US 2012/0131436 A1) relates to a system for automated report generation where entries in a report are linked to specific images, annotations, and a corresponding workflow state, allowing a user to resume a specific task by selecting a report entry.

Document D3 ("The caBIG(TM) Annotation and Image Markup Project") relates to the Annotation and Image Markup (AIM) project, which defines a standard information model and tools for creating structured, machine-readable annotations of medical images to overcome the limitations of free-text descriptions.

The following discloses certain improvements.

### SUMMARY

In one disclosed aspect, an image interpretation workstation comprises at least one display, at least one user input device, an electronic processor operatively connected with the at least one display and the at least one user input device, and a non-transitory storage medium storing instructions readable and executable by the electronic processor. Image interpretation environment instructions are readable and executable by the electronic processor to perform operations in accord with user inputs received via the at least one user input device including display of medical images on the at least one display, manipulation of displayed medical images, generation of finding objects, and construction of an image examination findings report. Finding object detection instructions are readable and executable by the electronic processor to detect generation of a finding object or user selection of a finding object via the at least one user input device. Patient record retrieval instructions are readable and executable by the electronic processor to identify and retrieve patient information relevant to a finding object detected by the finding object detection instructions from at least one electronic patient record. Patient record display instructions are readable and executable by the electronic processor to display patient information retrieved by the patient record retrieval instructions on the at least one display.

In another disclosed aspect, a non-transitory storage medium stores instructions readable and executable by an electronic processor operatively connected with at least one display and at least one user input device to perform an image interpretation method. The method comprises: providing an image interpretation environment to perform operations in accord user inputs received via the at least one user input device including display of medical images on the at least one display, manipulation of displayed medical images, generation of finding objects, and construction of an image examination findings report; monitoring the image interpretation environment to detect generation or user selection of a finding object; identifying and retrieving patient information relevant to the generated or user-selected finding object from at least one electronic patient record; and displaying the retrieved patient information on the at least one display and in the image interpretation environment.

In another disclosed aspect, an image interpretation method is performed by an electronic processor operatively connected with at least one display and at least one user input device. The image interpretation method comprises: providing an image interpretation environment to perform operations in accord user inputs received via the at least one user input device including display of medical images on the at least one display, manipulation of displayed medical images, generation of finding objects, and construction of an image examination findings report; monitoring the image interpretation environment to detect generation or user selection of a finding object; identifying and retrieving patient information relevant to the generated or user-selected finding object from at least one electronic patient record; and displaying the retrieved patient information on the at least one display and in the image interpretation environment.

One advantage resides in automatically providing patient record content relevant to an imaging finding in response to creation or selection of that finding.

Another advantage resides in providing an image interpretation workstation with an improved user interface.

Another advantage resides in providing an image interpretation workstation with more efficient retrieval of salient patient information.

Another advantage resides in providing contextual information related to a medical imaging finding.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The invention may take form in various components and arrangements of components, and in various steps and arrangements of steps. The drawings are only for purposes of illustrating the preferred embodiments and are not to be construed as limiting the invention.
FIGURE 1 diagrammatically illustrates an image interpretation workstation with automated retrieval of patient record information relevant to image findings.
FIGURES 2 and 3 present illustrative display examples suitably generated by the image interpretation workstation of FIGURE 1.
FIGURE 4 diagrammatically illustrates a process workflow for automated retrieval of patient record information relevant to image findings, which is suitably performed by the image interpretation workstation of FIGURE 1.

### DETAILED DESCRIPTION

It is recognized herein that existing approaches for integrating patient record information into the image interpretation workflow have certain deficiencies. For example, providing a separate, concurrently running patient record interface program does not provide timely access to relevant patient information. To use such a patient record interface, the radiologist, physician, ultrasound specialist, or other image interpreter must recognize that the patient record may contain relevant information at a given point in the image analysis, and must know *a priori* the specific patient information items that are most likely to be relevant, and must know where those items are located in the electronic patient record. As to the last point, the electronic patient record may be spread across a number of different databases, e.g. the Electronic Medical/Health Record (EMR or EHR) may store general patient information, the Cardiovascular Information System (CVIS) may store information specifically relating to cardiovascular care, the Picture Archiving and Communication Service (PACS) may store radiology images and related content such as radiology reports, and so forth. The particular database(s) organization is also likely to be specific to a particular hospital, which can be confusing for an image interpreter who practices at several different hospitals.

It is further recognized herein that the generation of a finding during the image interpretation (or, in some cases, the selection of a previously generated finding) can be leveraged to provide both a practical trigger for initiating the identification and retrieval of relevant patient information from the electronic patient record, and also the informational basis for such identification and retrieval. More particularly, some image interpretation workstations provide for automated or semi-automated generation of standardized and/or structured finding objects. In some radiology workstation environments, finding objects are generated in a standardized Annotation Image Mark-up (AIM) format. Similarly, some ultrasound image interpretation environments generate finding objects in the form of standardized finding codes (FCs), i.e. standard words or phrases expressing specific image findings. The generation or user selection of such a finding object is leveraged in embodiments herein to trigger a patient record retrieval operation, and the standardized and/or structured finding object provides the informational basis for this retrieval operation. The patient record retrieval process is preferably automatically triggered by generation or user selection of a finding object, and the standardized and/or structured finding object provides a finite space of data inputs so as to enable use of a relevant patient information look-up table that maps finding objects to patient information items, thereby enabling an automated retrieval process. Preferably, the retrieved patient information is automatically presented to the image interpreter in the (same) image interpretation environment being used by the image interpreter to perform the image interpretation process. In this way, the relevant patient information in the electronic patient record is automatically retrieved and presented to the image interpreter automatically, without any additional user interactions within the image interpretation environment, thus improving the user interface and operational efficiency of the image interpretation workstation.

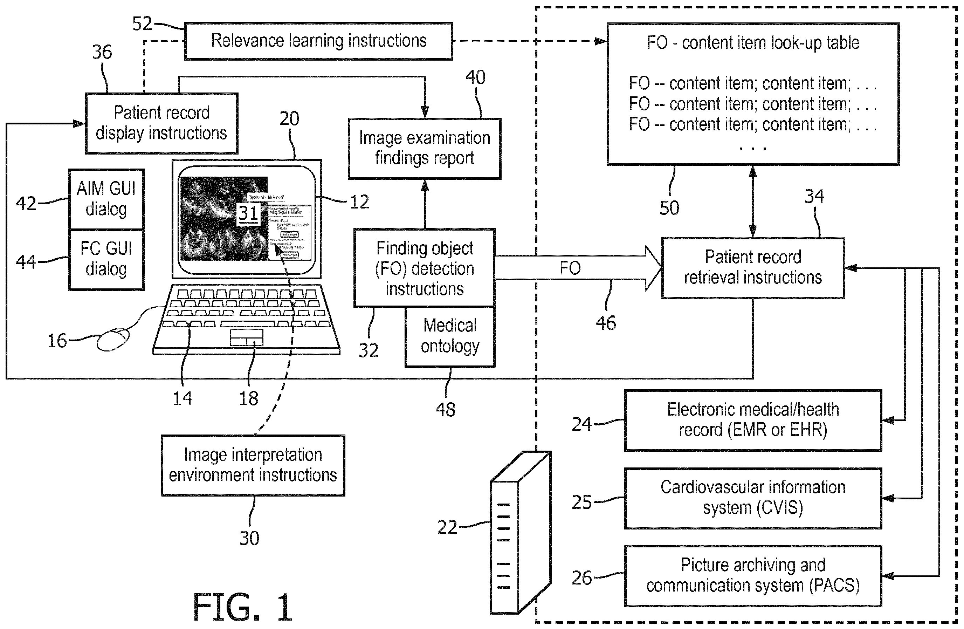

With reference to FIGURE 1, an illustrative image interpretation workstation includes at least one display **12** and at least one user input device, e.g. an illustrative keyboard **14;** an illustrative mouse **16,** trackpad **18,** trackball, touch-sensitive overlay of the display **12,** or other pointing device; a dictation microphone (not shown), or so forth. The illustrative image interpretation workstation further includes electronic processors **20, 22** - in the illustrative example, the electronic processor **20** is embodied for example as a local desktop or notebook computer (e.g., a local user interfacing computer)that is operated by the radiologist, ultrasound specialist, or image interpreter and include at least one microprocessor or microcontroller, and the electronic processer **22** is for example embodied as a remote server computer that is connected with the electronic processor **20** via a local area network (LAN), wireless local area network (WLAN), the Internet, various combinations thereof, and/or some other electronic data network. The electronic processor **22** optionally may itself include a plurality of interconnected computers, e.g. a computer cluster, a cloud computing resource, or so forth. The electronic processor **20, 22** includes or is in operative electronic communication with an electronic patient record **24, 25, 26,** which in the illustrative embodiment is distributed across several different databases: an Electronic Medical (or Health) Record (EMR or EHR) **24** which stores general patient information; a Cardiovascular Information System (CVIS) **25** which stores information specifically relating to cardiovascular care; and a Picture Archiving and Communication Service (PACS) **26** which stores radiology images. The electronic patient record **24, 25, 26** is just one exemplary embodiment of a patient record combination and may exclude one or more of the electronic patient records **24, 25, 26,** with various permutations or combinations of electronic patient records being possible, and with the electronic patient record constituting any number of databases or even a single database. The database(s) making up the electronic patient record may have different names than those of illustrative FIGURE 1, and may be specific to particular informational domains beside the illustrative general, cardiovascular, and radiology domains.

The image interpretation workstation further includes a non-transitory storage medium storing various instructions readable and executable by the electronic processor **20, 22** to perform various tasks. The non-transitory storage medium may, for example, comprise one or more of a hard disk drive or other magnetic storage medium, an optical disk or other optical storage medium, a solid state drive (SSD), FLASH memory, or other electronic storage medium, various combinations thereof, or so forth. In the illustrative embodiment, the non-transitory storage medium stores image interpretation environment instructions **30** which are readable and executable by the electronic processor **20, 22** to perform operations in accord with user inputs received via the at least one user input device **14, 16, 18** so as to implement an image interpretation environment **31.** These operations include display of medical images on the at least one display **12,** manipulation of displayed medical images, generation of finding objects, and construction of an image examination findings report. The image interpretation environment instructions **30** may implement substantially any suitable image interpretation environment **31,** for example a radiology reading environment, an ultrasound imaging interpretation environment, a combination thereof, or so forth. A radiology reading environment is typically operatively connected with the PACS 26 to retrieve images of radiology examinations and to enable entry of an image examination findings report, sometimes referred to as a radiology report in the radiology reading context. Similarly, for ultrasound image interpretation in the cardiovascular care context (e.g. echocardiogram acquisition), the image interpretation environment **31** is typically operatively connected with the CVIS **25** to retrieve echocardiogram examination images and to enable entry of an image examination findings report, which may be referred to as an echocardiogram report in this context.

To provide finding object-triggered automated access to relevant patient information stored in the electronic patient record **24, 25, 26,** as disclosed herein, the non-transitory storage medium also stores finding object detection instructions **32** which are readable and executable by the electronic processor **20, 22** to monitor the image interpretation environment **31** implemented by the image interpretation environment instructions **30** so as to detect generation of a finding object or user selection of a finding object via the at least one user input device **14, 16, 18.** The non-transitory storage medium also stores patient record retrieval instructions **34** which are readable and executable by the electronic processor **20, 22** to identify and retrieve patient information relevant to a finding object detected by the finding object detection instructions **32** from at least one electronic patient record **24, 25, 26.** Still further, the non-transitory storage medium stores patient record display instructions **36** which are readable and executable by the electronic processor **20, 22** to display patient information retrieved by the patient record retrieval instructions **34** on the at least one display **12** and in the image interpretation environment **31** implemented by the image interpretation environment instructions **30.**

In the following, some illustrative embodiments of these components are described in further detail.

The image interpretation environment instructions **30** implement the image interpretation environment **31** (e.g. a radiology reading environment, or an ultrasound image interpretation environment). The image interpretation environment **31** performs operations in accord with user inputs received via the at least one user input device **14, 16, 18** including display of medical images on the at least one display **12,** manipulation of displayed medical images (e.g. at least pan and zoom of displayed medical images, and optionally other manipulation such as applying a chosen image filter, adjusting the contrast function, contouring organs, tumors, or other image features, and/or so forth), and construction of an image examination findings report **40.** To construct the findings report **40,** the image interpretation environment **31** provides for the generation of findings.

More particularly, the image interpretation environment **31** provides for automated or semi-automated generation of standardized and/or structured finding objects. In some radiology workstation environments, finding objects are generated in a standardized Annotation Image Mark-up (AIM) format. In one contemplated user interface, the user selects an image location, such as a pixel of a computed tomography (CT), magnetic resonance (MR), or other radiology image, which is at or near a relevant finding (e.g., a tumor or aneurysm). This brings up a contextual AIM graphical user interface (GUI) dialog **42** (e.g. as a pop-up user dialog box), via which the image interpreter labels the finding with meta-data (i.e. an annotation) characterizing its size, morphology, enhancement, pathology, and/or so forth. These data form the finding object in AIM format. It is to be appreciated that AIM is an illustrative standard for encoding structured finding objects. Alternative standards for encoding structured finding objects are also contemplated. In general, the structured finding object preferably encodes "key-value" pairs specifying values for various data fields (keys), e.g. "anatomy = lung" is an illustrative example in AIM format, where "anatomy" is the key field and "lung" is the value field. In the AIM format, key-value pairs are hierarchically related through a defining XML standard. Other structured finding object formats can be used to similarly provide structure for representing finding objects, e.g. as key-value tuples of a suitably designed relational database table or the like (optionally with further columns representing attributes of the key field, et cetera).

In another illustrative example, suitable for an echocardiogram interpretation environment, the user interface responds to clicking a location on the image by bringing up a point-and-click finding code GUI dialog **44** via which the image interpreter can select the appropriate finding code, e.g. from a contextual drop-down list. Each finding code (FC) is a unique and codified observational or diagnostic statement about the cardiac anatomy, e.g. the finding code may be a word or phrase describing the anatomy feature.

The generation or user selection of the finding object is leveraged in embodiments disclosed herein to trigger a patient record retrieval operation, and the standardized and/or structured finding object provides the informational basis for this retrieval operation. The generation of a finding object (or, alternatively, the user selection of a previously created finding object) is detected by the FO detection instructions **32,** so as to generate a selected finding object (FO) **46.** The detection can be triggered, for example, by detecting the user operating a user input device **14, 16, 18** to close the FO generation (or editing) GUI dialog **42, 44.** Depending upon the particular format of the finding object, some conversion may optionally be performed to generate the FO **46** as a suitable informational element for searching the electronic patient record **24, 25, 26.** For example, in the case of a finding object in AIM or another structured format, a medical ontology **48** may be referenced to convert the finding object to a natural language word or phrase. For example, an ontology such as SNOMED or RadLex may be used for this purpose.

The patient record retrieval instructions **34** execute on the server computer **22** to receive the FO **46** and to use the informational content of the FO **46** to identify and retrieve patient information relevant to a FO **46** from at least one electronic patient record **24, 25, 26.** In one approach, a non-transitory storage medium stores a relevant patient information look-up table **50** that maps finding objects to patient information items. The look-up table **50** may be stored on the same non-transitory storage medium that stores some or all of the instructions **30, 32, 34, 36,** or may be stored on a different non-transitory storage medium. The term "information item" as used in this context refers to an identification of a database field, search term, or other locational information sufficient to enable the executing patient record retrieval instructions **34** to locate and retrieve certain relevant patient information. For example, if the FO indicates a lung tumor, relevant patient information may be whether the patient is a smoker or a non-smoker - accordingly, the look-up table for this FO may include the location of a database field in the EMR or EHR **24** containing that information. Similarly, the look-up table **50** may include an entry locating information on whether a histopathology examination has been performed to assess lung cancer, and/or so forth. As another example, in the case of the finding "Septum is thickened" in the context of an echocardiogram interpretation, the look-up table **50** may include the keywords "hypertrophic cardiomyopathy" and "diabetes" as these conditions are commonly associated with a thickened septum, and the electronic patient record **24, 25, 26** is searched for occurrences of these terms. If the content of the electronic patient record is codified using an ontology such as the International Classification of Diseases version 10 (ICD10), Current Procedural Terminology (CPT) or Systematized Nomenclature of Medicine (SNOMED), then these terms are suitably employed in the look-up table **50.** In such a case, the look-up table **50** may further include an additional column providing a natural language word or phrase description of the ICD-10 code or the like. A mapping is maintained between AIM-compliant objects and the history items, e.g., ICD10. The mapping provided by the look-up table **50** may for some entries involve partial objects, meaning that they need not be fully specified. A sample look-up table entry for radiology reading might be:
- Finding object: Anatomy = lung and morphology = nodule
- Relevant history: "F17 - Nicotine dependence"
while a sample look-up table entry for an echocardiogram interpretation mapping between FCs and the patient record items might be:
- Finding object: "Septum is thickened"
- Relevant history: "I42.2 - Hypertrophic cardiomyopathy"

The electronic patient record retrieval instructions **34** are executable by the electronic processor **22** to identify and retrieve patient information relevant to a finding object **46** by referencing the relevant patient information look-up table **50** for the locational information and then searching the electronic patient record **24, 25, 26** for relevant patient information at that location (e.g. specified as a specific database field, or as a search term to employ in a SQL query or the like, or so forth).

In a variant embodiment, a background mapping is deployed from FCs onto ontology concepts, as the FC are contained in an unstructured "flat" lexicon. Such a secondary mapping can be constructed manually or generated automatically using a concept extraction engine, e.g. MetaMap.

In the following, an illustrative implementation of the electronic patient record retrieval instructions **34** is described. The executing instructions **34** have access to one or more repositories of potentially heterogeneous medical documents and data. The Electronic Medical (or Health) Record (EMR or EHR) **24** is one instance of such a repository. The data sources can have multiple forms, for example: list of ICD10 codes (e.g., problem list, past medical history, allergies list); list of CPT codes (e.g., past surgery list); list of RxNorm codes (e.g., medication list); discrete numerical data elements (e.g., contained in lab reports and blood pressures); narrative documents (e.g., progress, surgery, radiology, pathology and operative reports); and/or so forth. With each clinical condition, zero or more dedicated search modules are defined, each searching one type of data source. For instance, with the clinical condition "diabetes" search modules can be associated that: match a list of known ICD10 diabetes codes against the patient's problem list; match a list of medications known to be associated with diabetes treatment (e.g., insulin) against the patient's medication list; match a glucose threshold against the patient's most recent lab report; match a list of key words (e.g., "diabetes", "DM2", "diabetic") in narrative progress reports; and/or so forth. If there are matches, the executing electronic patient record retrieval instructions **34** return pointers to the location(s) in the matching source document(s) as well as matching elements of information. In one implementation, the executing electronic patient record retrieval instructions **34** perform a free-text search based on a search query derived from the finding object **46,** e.g., "lower lobe lung nodule". This search can be implemented using various search methods, e.g., elastic search. If only FO-based text-based searches are employed, then the relevant history look-up table **50** is suitably omitted. In other embodiments, free-text searching using the words or phrases of the finding object **46** augments retrieval operations using the relevant history look-up table **50.**

The identified and retrieved patient history is displayed on the at least one display **12** by the executing patient record display instructions **36.** Preferably, the retrieved patient information is displayed on the at least one display **12** and in the image interpretation environment **31,** e.g. in a dedicated patient history window of the image interpretation environment **31,** as a pop-up window superimposed on a medical image displayed in the image interpretation environment **31,** or so forth. In this way, the user does not need to switch to a different application running on the electronic processor **20** (e.g., a separate electronic patient record interfacing application) in order to access the retrieved patient information, and this information is presented in the image interpretation environment **31** that the image interpreter is employing to view the medical images being interpreted. In some embodiments (described later below), relevance learning instructions **52** are readable and executable by the electronic processor **20, 22** to update the relevant patient information look-up table **50** by applying machine learning to user interactions with the displayed patient information via the at least one user input device **14, 16, 18.**

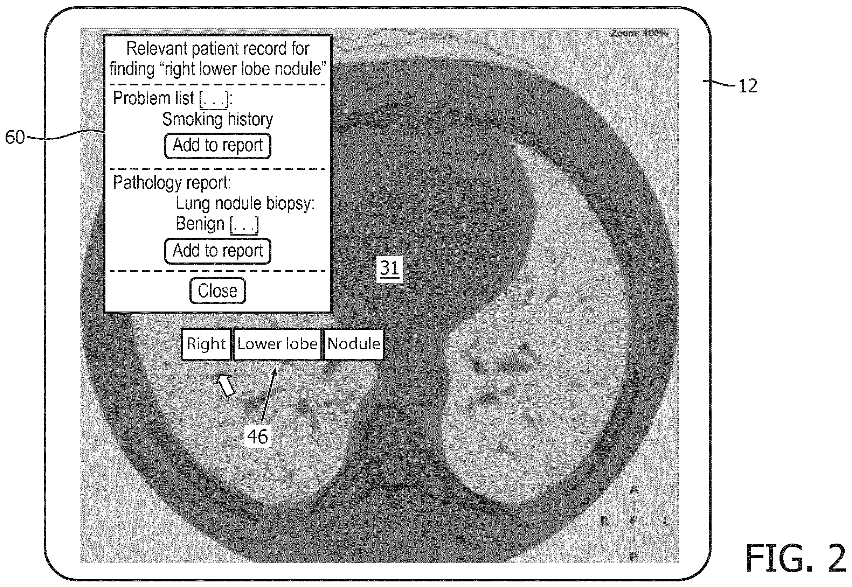

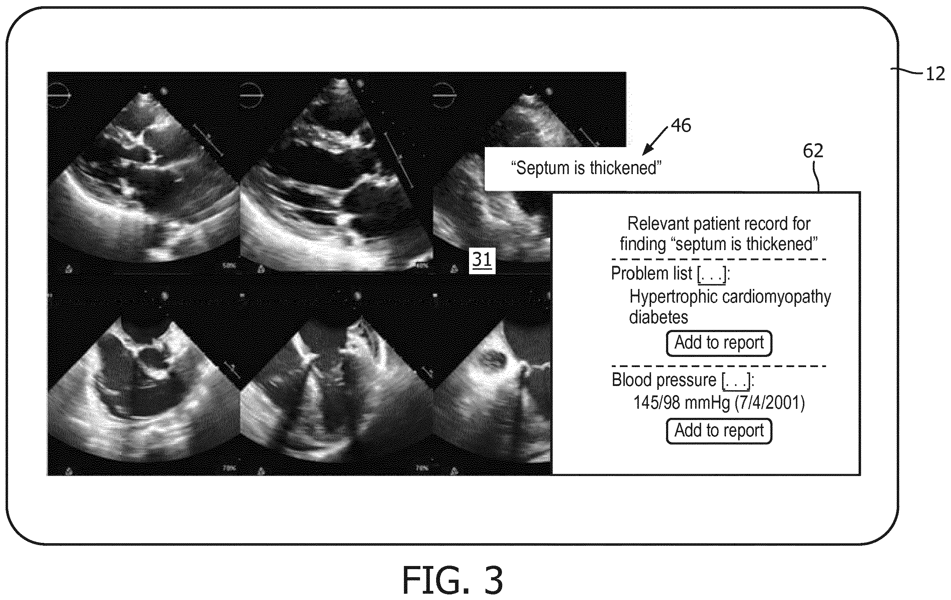

With reference to FIGURES 2 and 3, in some embodiments the executing patient record display instructions **36** are interactive, e.g., by clicking on a particular piece of displayed patient information, a panel appears that displays the source document (e.g., the narrative report) highlighting the matching information and its surrounding content. FIGURE 2 illustrates a contemplated display in which the image interpretation environment **31** is a radiology reading environment. The finding object **46** in this example is "right lower lobe nodule") and is created (e.g. using the AIM GUI dialog **42,** or more generally a GUI dialog for entering the finding in another structured format so as to create a structured finding object) and detected by the executing FO detection instructions **32** which monitor the image interpretation environment **31** for generation or user selection of FOs. This detection of the FO **46** triggers execution of the patient record retrieval instructions **34,** and the retrieval of relevant patient information then triggers execution of the patient record display instructions **36** to display of relevant clinical history in a pop-up window **60** in the illustrative example of FIGURE 2. The underlined elements [...] shown in the window **60** indicate dynamic hyperlinks that will open up the source document centered at the matching information. Additionally, the window **60** includes "Add to report" buttons which can be clicked to add the corresponding patient information to the image examination finding report **40** (see FIGURE 1). A "Close" button in the window **60** can be clicked to close the patient information window **60.**

FIGURE 3 illustrates a contemplated display in which the image interpretation environment **31** is an echocardiogram image interpretation environment. The finding object **46** in this example is "Septum is thickened" and is created (e.g. using the FC GUI dialog **44)** and detected by the executing FO detection instructions **32** which monitor the image interpretation environment **31** for generation or user selection of FOs. This detection of the FO **46** triggers execution of the patient record retrieval instructions **34,** and the retrieval of relevant patient information then triggers execution of the patient record display instructions **36** to display of relevant clinical history in a separate patient information window **62** of the image interpretation environment **31.** The underlined elements [...] shown in the window **62** indicate dynamic hyperlinks that will open up the source document centered at the matching information. Additionally, the window **62** includes "Add to report" buttons which can be clicked to add the corresponding patient information to the image examination finding report **40** (see FIGURE 1). A "Close" button in the window **62** can be clicked to close the patient information window **62.**

With returning reference to FIGURE 1, the relevance learning instructions **52** are readable and executable by the electronic processor **20, 22** to update the relevant patient information look-up table **50** by applying machine learning to user interactions with the displayed patient information via the at least one user input device **14, 16, 18.** For example, if the user clicks on one of the "Add to report" buttons in the window **60** of FIGURE 2 (or on one of the "Add to report" buttons in the window **62** of FIGURE 3), this may be taken as an indication that the image interpreter has concluded the corresponding patient information that is added to the image examination findings report **40** was indeed relevant in the view of the image interpreter. By contrast, any piece of patient information which is not added to the report **40** by selection of its corresponding "Add to report" button was presumably not deemed to be relevant by the image interpreter. These user interactions therefore enable the pieces of patient information to be labeled as "relevant" (if the corresponding "Add to report" button is clicked) or "not relevant" (if the corresponding "Add to report" button is not clicked), and these labels can then be treated as human annotations, e.g. as ground-truth values. The executing relevance learning instructions **52** then update the relevant patient information look-up table **50** by applying machine learning to these user interactions, e.g. by removing any entry of the look-up table **50** that produces pieces of patient information whose "relevant"-to-"not relevant" ratio is below some threshold based on the interactions. To enable addition of new information, it is contemplated to statistically add additional information in the patient information retrieval that is not part of the look-up table **50** - if these additions are selected by the user as "relevant" with statistics above a certain threshold then they may be added to the look-up table **50.** These are merely illustrative examples, and other machine learning approaches may be used.

In the illustrative embodiment, execution of the various executable instructions **30, 32, 34, 36** is distributed between the local workstation computer **20** and the remote server computer **22.** Specifically, in the illustrative embodiment the image interpretation environment instructions **30,** the finding object detection instructions **32,** and the patient record display instructions **36** are executed locally by the local workstation computer **20;** whereas, the patient record retrieval instructions **34** are executed remotely by the remote server computer **22.** This is merely an illustrative example, and the instructions execution may be variously distributed amongst two or more provided electronic processors **20, 22,** or there may be a single electronic processor that performs all instructions.

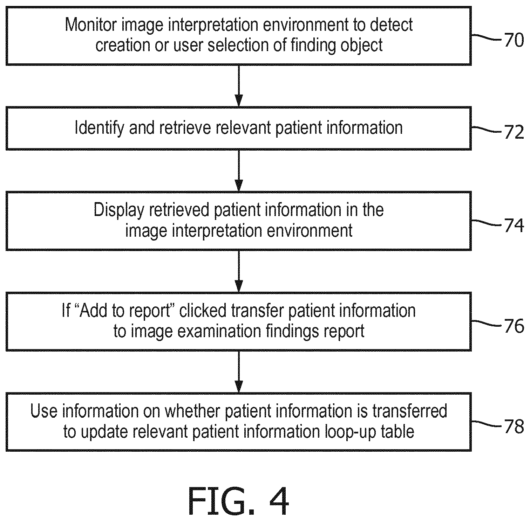

With reference to FIGURE 4 and continuing reference to FIGURE 1, an illustrative image interpretation method suitably performed by the image interpretation workstation of FIGURE 1 is described. In an operation **70,** the image interpretation environment **31** is monitored to detect creation or user selection of a finding object **46.** In an operation **72,** the relevant patient information is identified and retrieved from the electronic patient record **24, 25, 26,** e.g. using the relevant patient information look-up table **50.** In an operation **74,** the retrieved relevant patient information is displayed in the image interpretation environment **31.** In an optional operation **76,** if the patient clicks on the "Add to report" button in the window **60, 62** of respective FIGURES 2 and 3, or otherwise selects to add a piece of retrieved patient information to the image examination findings report **40,** then this information is added to the report **40.** In an optional operation **78,** the user interaction data on which pieces of retrieved patient information are actually added to the image examination findings report **40** is processed by machine learning to update the relevant patient information look-up table **50.**

The invention has been described with reference to the preferred embodiments. Modifications and alterations may occur to others upon reading and understanding the preceding detailed description. It is intended that the invention be construed as including all such modifications and alterations insofar as they come within the scope of the appended claims or the equivalents thereof.

## Claims

1. An image interpretation workstation comprising:
at least one display (12);
at least one user input device (14, 16, 18); an electronic processor (20, 22) operatively connected with the at least one display and the at least one user input device storing:
image interpretation environment instructions (30) readable and executable by the electronic processor to perform operations in accord with user inputs received via the at least one user input device including display of medical images on the at least one display, manipulation of displayed medical images, generation of finding objects, and construction of an image examination findings report (40);
finding object detection instructions (32) readable and executable by the electronic processor to detect generation of a finding object or user selection of a finding object via the at least one user input device, wherein the finding object is a clinical finding object comprising a finding annotation or finding code expressing a specific image finding;
patient record retrieval instructions (34) readable and executable by the electronic processor to identify and retrieve patient information relevant to a finding object (40) detected by the finding object detection instructions from at least one electronic patient record (24, 25, 26);
patient record display instructions (36) readable and executable by the electronic processor to display patient information retrieved by the patient record retrieval instructions on the at least one display, wherein execution of the patient record retrieval instructions (34) is triggered by detection of generation or user selection of a finding object (46) by the executing finding object detection instructions (34) and wherein execution of the patient record display instructions (36) is triggered by retrieval of patient information relevant to a finding object by the executing patient record retrieval instructions;
wherein the patient record retrieval instructions (34) are executable by the electronic processor (20, 22) to identify and retrieve patient information relevant to a finding object (46) by referencing a relevant patient information look-up table that maps finding objects to patient information items and/or by performing a free-text search based on a search query derived from the finding object.

2. The image interpretation workstation of claim 1, , whereinrelevance learning instructions (52) readable and executable by the electronic processor (20, 22) to update the relevant patient information look-up table (50) by applying machine learning to user interactions with the displayed patient information via the at least one user input device (14, 16, 18).

3. The image interpretation workstation of any one of claim 1 or 2, wherein the patient record display instructions (36) are further readable and executable by the electronic processor (20, 22) to detect selection via the at least one user input device (14, 16, 18) of displayed patient information retrieved by the patient record retrieval instructions (34) and to display a corresponding source document retrieved from at least one electronic patient record (24, 25, 26).

4. The image interpretation workstation of any one of claims 1-3, wherein:
the image interpretation environment instructions (30) are executable to generate finding objects in a structured format by operations including user selection of an image feature of a displayed medical image via the at least one user input device (14, 16, 18) and input of at least one finding annotation via interaction of the at least one user input device with a graphical user interface dialog (42); and
the finding object detection instructions (32) are executable by the electronic processor (20, 22) to detect a finding object in the structured format and to convert the finding object in the structured format to a natural language word or phrase using a medical ontology (48).

5. The image interpretation workstation of any one of claims 1-3, wherein:
the image interpretation environment instructions (30) are executable to generate a finding object by user selection of a finding code via interaction of the at least one user input device (14, 16, 18) with a graphical user interface dialog (44); and
the finding object detection instructions are executable by the electronic processor (20, 22) to detect generation or user selection of a finding code.

6. The image interpretation workstation of any one of claims 1-5, wherein:
the patient record display instructions (36) are further readable and executable by the electronic processor (20, 22) to copy patient information retrieved by the patient record retrieval instructions (34) to the image examination findings report (40) in accord with user inputs received via the at least one user input device (14, 16, 18).

7. The image interpretation workstation of any one of claims 1-6, wherein the image interpretation environment instructions (30) are executable to perform manipulation including at least pan and zoom of displayed medical images in accord with user inputs received via the at least one user input device (14, 16, 18).

8. A computer-implemented method for performing an image interpretationcomprising the steps of:
providing an image interpretation environment (31) to perform operations in accord user inputs received via the at least one user input device including display of medical images on the at least one display, manipulation of displayed medical images, generation of finding objects, and construction of an image examination findings report (40);
monitoring the image interpretation environment to detect generation or user selection of a finding object (46), wherein the finding object is a clinical finding object comprising a finding annotation or finding code expressing a specific image finding;
identifying and retrieving patient information relevant to the generated or user-selected finding object from at least one electronic patient record (24, 25, 26), wherein said identifying and is triggered by detection of the generated or user selected finding object (46); and
displaying the retrieved patient information on the at least one display and in the image interpretation environment, wherein said displaying of the retrieved patient information is triggered by the retrieval of the patient information;
wherein the identifying and retrieving of patient information operates by referencing a relevant patient information look-up table that maps finding objects to patient information items and/or by performing a free-text search based on a search query derived from the finding object.

9. The method according to claim 8, further comprising:
updating the relevant patient information look-up table (50) by applying machine learning to user interactions with the displayed patient information via the at least one user input device (14, 16, 18).

10. The method of any one of claim 8 or 9, further comprising:
detecting selection via the at least one user input device (14, 16, 18) of selected displayed patient information; and
displaying a source document retrieved from at least one electronic patient record and containing the selected displayed patient information.

11. The method of any one of claims 8-10, wherein:
the image interpretation environment (31) generates finding objects in an Annotation Image Mark-up format by operations including user selection of an image feature of a displayed medical image via the at least one user input device (14, 16, 18) and input of at least one finding annotation via the at least one user input device interacting with a graphical user interface dialog (42); and
the monitoring of the image interpretation environment to detect generation or user selection of a finding object (46) includes detecting a finding object in the Annotation Image Mark-up format and converting the finding object in the Annotation Image Mark-up format to a natural language word or phrase using a medical ontology (48).

12. The method of any one of claims 8-11, wherein:
the image interpretation environment (31) generates finding objects by user selection of a finding code via interaction of the at least one user input device (14, 16, 18) with a graphical user interface dialog (44); and
the monitoring of the image interpretation environment to detect generation or user selection of a finding object (46) includes detecting generation or user selection of a finding code.

13. The method of any one of claims 8-12, wherein the manipulation of displayed medical images provided by the image interpretation environment (31) includes at least pan and zoom of displayed medical images in accord with user inputs received via the at least one user input device (14, 16, 18).

14. A computer program product comprising instructions which, when the program is executed by a computer, cause the computer to carry out the steps of any one of claims 8-13.

## Patentansprüche

1. Bildinterpretations-Workstation, umfassend:
mindestens eine Anzeige (12);
mindestens eine Benutzereingabevorrichtung (14, 16, 18);
einen elektronischen Prozessor (20, 22), der betriebsfähig mit der mindestens einen Anzeige verbunden ist, und wobei die mindestens eine Benutzereingabevorrichtung Folgendes speichert:
Anweisungen (30) für eine Bildinterpretationsumgebung, die vom elektronischen Prozessor gelesen und ausgeführt werden können, um Operationen gemäß über die mindestens eine Benutzereingabevorrichtung empfangener Benutzereingaben durchzuführen, die Anzeige von medizinischen Bildern auf der mindestens einen Anzeige, Manipulation der angezeigten medizinischen Bilder, Erzeugung von Befundobjekten und Erstellung eines Berichts (40) mit Ergebnissen der Bilduntersuchung einschließen;
Anweisungen (32) zum Erkennen von Befundobjekten, die vom elektronischen Prozessor gelesen und ausgeführt werden können, um Erzeugung eines Befundobjekts oder Benutzerauswahl eines Befundobjekts über die mindestens eine Benutzereingabevorrichtung zu erkennen, wobei es sich bei dem Befundobjekt um ein klinisches Befundobjekt handelt, das eine Befundannotation oder einen Befundcode umfasst, die einen spezifischen Bildbefund ausdrücken;
Anweisungen (34) zum Abrufen von Patientenakten, die vom elektronischen Prozessor gelesen und ausgeführt werden können, um Patienteninformationen aus mindestens einer elektronischen Patientenakte (24, 25, 26) zu identifizieren und abzurufen, die für ein durch die Anweisungen zum Erkennen von Befundobjekten erkanntes Befundobjekt (40) relevant sind;
Anweisungen (36) zum Anzeigen von Patientenakten, die vom elektronischen Prozessor gelesen und ausgeführt werden können, um von den Anweisungen zum Abrufen von Patientenakten abgerufene Patientenakten auf der mindestens einen Anzeige anzuzeigen, wobei Ausführung der Anweisungen (34) zum Abrufen von Patientenakten durch Erkennen von Erzeugung oder Benutzerauswahl eines Befundobjekts (46) durch Ausführen der Anweisungen (34) zum Abrufen von Patientenakten ausgelöst wird, und wobei Ausführen der Anweisungen (36) zum Anzeigen von Patientenakten durch Abrufen von Patientenakten ausgelöst wird, die für ein Befundobjekt relevant sind, durch Ausführen der Anweisungen zum Abrufen von Patientenakten;
wobei die Anweisungen (34) zum Abrufen von Patientenakten vom elektronischen Prozessor (20, 22) ausgeführt werden können, um Patienteninformationen zu identifizieren und abzurufen, die für ein Befundobjekt (46) relevant sind, unter Bezug auf eine relevante Patienteninformations-Nachschlagetabelle, die Befundobjekte zu Patienteninformationselementen zuordnet und/oder durch Durchführen einer Freitextsuche basierend auf einer Suchanfrage, die aus dem Befundobjekt abgeleitet ist.

2. Bildinterpretations-Workstation nach Anspruch 1, wobei Relevanzlernanweisungen (52) vom elektronischen Prozessor (20, 22) gelesen und ausgeführt werden können, um die relevante Patienteninformations-Nachschlagetabelle (50) durch Anwendung von maschinellem Lernen auf Benutzerinteraktionen mit den angezeigten Patienteninformationen über die mindestens eine Benutzereingabevorrichtung (14, 16, 18) zu aktualisieren.

3. Bildinterpretations-Workstation nach einem der Ansprüche 1 oder 2, wobei die Anweisungen (36) zum Anzeigen von Patientenakten vom elektronischen Prozessor (20, 22) weiter gelesen und ausgeführt werden können, um Auswahl von über die mindestens eine Benutzereingabevorrichtung (14, 16, 18) angezeigten Patienteninformationen zu erkennen, die durch die Anweisungen (34) zum Abrufen von Patientenakten abgerufen wurden, und ein entsprechendes Quelldokument anzuzeigen, das aus mindestens einer elektronischen Patientenakte (24, 25, 26) abgerufen wurde.

4. Bildinterpretations-Workstation nach einem der Ansprüche 1-3, wobei:
die Anweisungen (30) für eine Bildinterpretationsumgebung ausgeführt werden können, um durch Operationen, die Benutzerauswahl eines Bildmerkmals eines angezeigten medizinischen Bildes über die mindestens eine Benutzereingabevorrichtung (14, 16, 18) und Eingabe mindestens einer Befundannotation durch Interaktion der mindestens einen Benutzereingabevorrichtung mit einem Dialog einer grafischen Benutzeroberfläche (42) einschließen, Befundobjekte in einem strukturierten Format zu erzeugen; und
die Anweisungen (32) zum Erkennen von Befundobjekten vom elektronischen Prozessor (20, 22) ausgeführt werden können, um ein Befundobjekt im strukturierten Format zu erkennen und das Befundobjekt im strukturierten Format unter Verwendung einer medizinischen Ontologie (48) in ein Wort oder eine Phrase natürlicher Sprache umzuwandeln.

5. Bildinterpretations-Workstation nach einem der Ansprüche 1-3, wobei:
die Anweisungen (30) für eine Bildinterpretationsumgebung ausgeführt werden können, um ein Befundobjekt durch Benutzerauswahl eines Befundcodes durch Interaktion der mindestens einen Benutzereingabevorrichtung (14, 16, 18) mit einem Dialog einer grafischen Benutzeroberfläche (44) zu erzeugen; und
die Anweisungen zum Erkennen von Befundobjekten vom elektronischen Prozessor (20, 22) ausgeführt werden können, um Erzeugung oder Auswahl eines Befundcodes zu erkennen.

6. Bildinterpretations-Workstation nach einem der Ansprüche 1-5, wobei:
die Anweisungen (36) zum Anzeigen von Patientenaktenweiter vom elektronischen Prozessor (20, 22) gelesen und ausgeführt werden können, um Patienteninformationen, die durch die Anweisungen (34) zum Abrufen von Patientenakten abgerufen wurden, gemäß über die mindestens eine Benutzereingabevorrichtung (14, 16, 18) empfangene Benutzereingaben in den Bericht (40) mit Ergebnissen der Bilduntersuchung zu kopieren.

7. Bildinterpretations-Workstation nach einem der Ansprüche 1-6, wobei die Anweisungen (30) für eine Bildinterpretationsumgebung ausgeführt werden können, um Manipulationen durchzuführen, die mindestens Schwenken und Zoomen der angezeigten medizinischen Bilder gemäß über die mindestens eine Benutzereingabevorrichtung (14, 16, 18) empfangene Benutzereingaben einschließen.

8. Computerimplementiertes Verfahren zum Durchführen einer Bildinterpretation, das die folgenden Schritte umfasst:
Bereitstellen einer Bildinterpretationsumgebung (31) zum Durchführen von Operationen gemäß über die mindestens eine Benutzereingabevorrichtung empfangener Benutzereingaben, die Anzeigen medizinischer Bilder auf der mindestens einen Anzeige, Manipulation von angezeigten medizinischen Bildern, Erzeugung von Befundobjekten und Erstellung eines Berichts (40) mit Ergebnissen der Bilduntersuchung einschließen;
Überwachen der Bildinterpretationsumgebung, um Erzeugung oder Benutzerauswahl eines Befundobjekts (46) zu erkennen, wobei es sich bei dem Befundobjekt um ein klinisches Befundobjekt handelt, das eine Befundannotation oder einen Befundcode umfasst, die einen spezifischen Bildbefund ausdrücken;
Identifizieren und Abrufen aus mindestens einer elektronischen Patientenakte (24, 25, 26) von Patienteninformationen, die für das erzeugte oder vom Benutzer ausgewählte Befundobjekt relevant sind, wobei das Identifizieren durch Erkennen des erzeugten oder vom Benutzer ausgewählten Befundobjekts (46) ausgelöst wird; und
Anzeigen der abgerufenen Patienteninformationen auf der mindestens einen Anzeige und in der Bildinterpretationsumgebung, wobei das Anzeigen der abgerufenen Patienteninformationen durch den Abruf der Patienteninformationen ausgelöst wird;
wobei das Identifizieren und Abrufen von Patienteninformationen unter Bezug auf eine relevante Patienteninformations-Nachschlagetabelle erfolgt, die Befundobjekte zu Patienteninformationselementen zuordnet und/oder durch Durchführen einer Freitextsuche basierend auf einer Suchanfrage, die aus dem Befundobjekt abgeleitet ist.

9. Verfahren nach Anspruch 8, weiter umfassend:
Aktualisierung der relevanten Patienteninformations-Nachschlagetabelle (50) durch Anwendung von maschinellem Lernen auf Benutzerinteraktionen mit den angezeigten Patienteninformationen über die mindestens eine Benutzereingabevorrichtung (14, 16, 18).

10. Verfahren nach einem von Anspruch 8 oder 9, weiter umfassend:
Erkennen von Auswahl von angezeigten Patienteninformationen über die mindestens eine Benutzereingabevorrichtung (14, 16, 18); und
Anzeigen eines Quelldokuments, das aus mindestens einer elektronischen Patientenakte abgerufen wurde und die ausgewählten angezeigten Patienteninformationen enthält.

11. Verfahren nach einem der Ansprüche 8-10, wobei:
die Bildinterpretationsumgebung (31) Befundobjekte im Annotations-Bild-Markup-Format erzeugt durch Operationen, die Benutzerauswahl eines Bildmerkmals eines angezeigten medizinischen Bildes über die mindestens eine Benutzereingabevorrichtung (14, 16, 18) und Eingabe mindestens einer Befundannotation über die mindestens eine Benutzereingabevorrichtung, die mit einem Dialog einer grafischen Benutzeroberfläche (42) interagiert, einschließt; und
Überwachen der Bildinterpretationsumgebung zum Erkennen von Erzeugung oder Benutzerauswahl eines Befundobjekts (46) Erkennen eines Befundobjekts im Annotations-Bild-Markup-Format und Umwandeln des Befundobjekts im Annotations-Bild-Markup-Format in ein Wort oder eine Phrase natürlicher Sprache unter Verwendung einer medizinischen Ontologie (48) einschließt.

12. Verfahren nach einem der Ansprüche 8-11, wobei:
die Bildinterpretationsumgebung (31) Befundobjekte erzeugt durch Benutzerauswahl eines Befundcodes durch Interaktion der mindestens einen Benutzereingabevorrichtung (14, 16, 18) mit einem Dialog einer grafischen Benutzeroberfläche (44); und
Überwachen der Bildinterpretationsumgebung zum Erkennen von Erzeugung oder Benutzerauswahl eines Befundobjekts (46) Erkennen von Erzeugung oder Benutzerauswahl eines Befundcodes einschließt.

13. Verfahren nach einem der Ansprüche 8-12, wobei die Manipulation der von der Bildinterpretationsumgebung (31) bereitgestellten angezeigten medizinischen Bilder mindestens Schwenken und Zoomen der angezeigten medizinischen Bilder gemäß über das mindestens eine Benutzereingabevorrichtung (14, 16, 18) empfangene Benutzereingaben einschließt.

14. Computerprogrammprodukt, das Anweisungen umfasst, die, wenn das Programm von einem Computer ausgeführt wird, bewirken, dass der Computer das Verfahren nach einem der Ansprüche 8-13 ausführt.

## Revendications

1. Poste de travail d'interprétation d'image, comprenant :
au moins un dispositif d'affichage (12) ;
au moins un dispositif d'entrée d'utilisateur (14, 16, 18) ;
un processeur électronique (20, 22) connecté fonctionnellement à l'au moins un dispositif d'affichage et à l'au moins un dispositif d'entrée d'utilisateur stockant :
des instructions d'environnement d'interprétation d'image (30) pouvant être lues et exécutées par le processeur électronique pour effectuer des opérations conformément à des entrées d'utilisateur reçues via au moins un dispositif d'entrée d'utilisateur, y compris un affichage d'images médicales sur le au moins un dispositif d'affichage, une manipulation d'images médicales affichées, une génération d'objets de recherche et une construction d'un rapport de recherches d'examen d'image (40) ;
des instructions de détection d'objet de recherche (32) pouvant être lues et exécutées par le processeur électronique pour détecter une génération d'un objet de recherche ou une sélection d'utilisateur d'un objet de recherche via au moins un dispositif d'entrée d'utilisateur, dans lequel l'objet de recherche est un objet de recherche clinique comprenant une annotation de recherche ou un code de recherche exprimant une recherche d'image spécifique ;
des instructions de récupération de dossier de patient (34) pouvant être lues et exécutées par le processeur électronique pour identifier et récupérer des informations de patient pertinentes pour un objet de recherche (40) détecté par les instructions de détection d'objet de recherche à partir d'au moins un dossier de patient électronique (24, 25, 26) ;
des instructions d'affichage de dossier de patient (36) pouvant être lues et exécutées par le processeur électronique pour afficher des informations de patient récupérées par les instructions de récupération de dossier de patient sur le au moins un dispositif d'affichage, dans lequel l'exécution des instructions de récupération de dossier de patient (34) est déclenchée par la détection de la génération ou de la sélection d'utilisateur d'un objet de recherche (46) par l'exécution des instructions de détection d'objet de recherche (34) et dans lequel l'exécution des instructions d'affichage de dossier de patient (36) est déclenchée par la récupération d'informations de patient pertinentes pour un objet de recherche par l'exécution des instructions de récupération de dossier de patient ;
dans lequel les instructions de récupération de dossier de patient (34) peuvent être exécutées par le processeur électronique (20, 22) pour identifier et récupérer des informations de patient pertinentes pour un objet de recherche (46) par référence à une table de consultation d'informations de patient pertinentes qui associe des objets de recherche à des éléments d'informations de patient et/ou en effectuant une recherche en texte libre basée sur une requête de recherche dérivée de l'objet trouvé.

2. Poste de travail d'interprétation d'image selon la revendication 1, dans lequel des instructions d'apprentissage de pertinence (52) pouvant être lues et exécutées par le processeur électronique (20, 22) mettent à jour la table de recherche d'informations de patient pertinentes (50) en appliquant un apprentissage automatique aux interactions d'utilisateur avec les informations de patient affichées via au moins un dispositif d'entrée d'utilisateur (14, 16, 18).

3. Poste de travail d'interprétation d'image selon l'une quelconque des revendications 1 ou 2, dans lequel les instructions d'affichage de dossier de patient (36) peuvent en outre être lues et exécutées par le processeur électronique (20, 22) pour détecter une sélection via au moins un dispositif d'entrée d'utilisateur (14, 16, 18) d'informations de patient affichées récupérées par les instructions de récupération de dossier de patient (34) et pour afficher un document source correspondant récupéré à partir d'au moins un dossier de patient électronique (24, 25, 26).

4. Poste de travail d'interprétation d'image selon l'une quelconque des revendications 1-3, dans lequel :
les instructions d'environnement d'interprétation d'image (30) peuvent être exécutées pour générer des objets de recherche dans un format structuré par des opérations incluant une sélection d'utilisateur d'une caractéristique d'image d'une image médicale affichée via le au moins un dispositif d'entrée d'utilisateur (14, 16, 18) et l'entrée d'au moins une annotation de recherche via une interaction du au moins un dispositif d'entrée d'utilisateur avec une boîte de dialogue d'interface utilisateur graphique (42) ; et
les instructions de détection d'objet de recherche (32) peuvent être exécutées par le processeur électronique (20, 22) pour détecter un objet de recherche au format structuré et pour convertir l'objet de recherche au format structuré en un mot ou une phrase en langage naturel à l'aide d'une ontologie médicale (48).

5. Poste de travail d'interprétation d'image selon l'une quelconque des revendications 1-3, dans lequel :
les instructions d'environnement d'interprétation d'image (30) peuvent être exécutées pour générer un objet de recherche par une sélection d'utilisateur d'un code de recherche via une interaction du au moins un dispositif d'entrée d'utilisateur (14, 16, 18) avec une boîte de dialogue d'interface utilisateur graphique (44) ; et
les instructions de détection d'objet de recherche peuvent être exécutées par le processeur électronique (20, 22) pour détecter une génération ou une sélection d'utilisateur d'un code de recherche.

6. Poste de travail d'interprétation d'image selon l'une quelconque des revendications 1-5, dans lequel :
les instructions d'affichage de dossier de patient (36) peuvent en outre être lues et exécutées par le processeur électronique (20, 22) pour copier les informations de patient récupérées par les instructions de récupération de dossier de patient (34) dans le rapport de recherches d'examen d'image (40) conformément à des entrées d'utilisateur reçues via le au moins un dispositif d'entrée d'utilisateur (14, 16, 18).

7. Poste de travail d'interprétation d'image selon l'une quelconque des revendications 1-6, dans lequel les instructions d'environnement d'interprétation d'image (30) peuvent être exécutées pour effectuer une manipulation incluant au moins un panoramique et un zoom d'images médicales affichées conformément à des entrées d'utilisateur reçues via le au moins un dispositif d'entrée d'utilisateur (14, 16, 18).

8. Procédé mis en œuvre par ordinateur pour effectuer une interprétation d'image comprenant les étapes de :
fourniture d'un environnement d'interprétation d'image (31) pour effectuer des opérations conformément à des entrées d'utilisateur reçues via au moins un dispositif d'entrée d'utilisateur, incluant un affichage d'images médicales sur le au moins un dispositif d'affichage, une manipulation d'images médicales affichées, une génération d'objets de recherche et une construction d'un rapport de recherches d'examen d'image (40) ;
surveillance de l'environnement d'interprétation d'image pour détecter une génération ou une sélection d'utilisateur d'un objet de recherche (46), dans lequel l'objet de recherche est un objet de recherche clinique comprenant une annotation de recherche ou un code de recherche exprimant une recherche d'image spécifique ;
identification et récupération des informations de patient pertinentes pour l'objet de recherche généré ou sélectionné par l'utilisateur à partir d'au moins un dossier de patient électronique (24, 25, 26), dans lequel ladite identification ladite récupération sont déclenchées par une détection de l'objet de recherche généré ou sélectionné par l'utilisateur (46) ; et
affichage des informations de patient récupérées sur le au moins un dispositif d'affichage et dans l'environnement d'interprétation d'image, dans lequel ledit affichage des informations de patient récupérées est déclenché par la récupération des informations de patient ;
dans lequel l'identification et la récupération d'informations de patient interviennent par référence à une table de consultation d'informations de patient pertinentes qui cartographie des objets de recherche par rapport aux éléments d'information de patient et/ou en effectuant une recherche en texte libre basée sur une requête de recherche dérivée de l'objet de recherche.

9. Procédé selon la revendication 8, comprenant en outre :
la mise à jour de la table de consultation d'informations de patient pertinentes (50) en appliquant un apprentissage automatique à des interactions d'utilisateur avec les informations de patient affichées via le au moins un dispositif d'entrée d'utilisateur (14, 16, 18).

10. Procédé selon l'une quelconque des revendications 8 ou 9, comprenant en outre :
la détection d'une sélection via le au moins un dispositif d'entrée d'utilisateur (14, 16, 18) d'informations de patient affichées sélectionnées ; et
l'affichage d'un document source récupéré à partir d'au moins un dossier de patient électronique et contenant les informations de patient affichées sélectionnées.

11. Procédé selon l'une quelconque des revendications 8-10, dans lequel
l'environnement d'interprétation d'image (31) génère des objets de recherche au format AIM (Annotation Image Mark-up) par des opérations incluant une sélection d'utilisateur d'une caractéristique d'image d'une image médicale affichée via le au moins un dispositif d'entrée d'utilisateur (14, 16, 18) et l'entrée d'au moins une annotation de recherche via le au moins un dispositif d'entrée d'utilisateur interagissant avec une boîte de dialogue d'interface utilisateur graphique (42) ; et
la surveillance de l'environnement d'interprétation d'image pour détecter une génération ou une sélection d'utilisateur d'un objet de recherche (46) inclut une détection d'un objet de recherche au format AIM (Annotation Image Mark-up) et la conversion de l'objet de recherche au format AIM (Annotation Image Mark-up) en un mot ou une phrase en langage naturel à l'aide d'une ontologie médicale (48).

12. Procédé selon l'une quelconque des revendications 8-11, dans lequel :
l'environnement d'interprétation d'image (31) génère des objets de recherche par une sélection d'utilisateur d'un code de recherche via l'interaction du au moins un dispositif d'entrée d'utilisateur (14, 16, 18) avec une boîte de dialogue d'interface utilisateur graphique (44) ; et
la surveillance de l'environnement d'interprétation d'image pour détecter une génération ou une sélection d'utilisateur d'un objet de recherche (46) inclut une détection d'une génération ou d'une sélection d'utilisateur d'un code de recherche.

13. Procédé selon l'une quelconque des revendications 8-12, dans lequel la manipulation d'images médicales affichées fournies par l'environnement d'interprétation d'image (31) inclut au moins un panoramique et un zoom d'images médicales affichées conformément à des entrées d'utilisateur reçues via le au moins un dispositif d'entrée d'utilisateur (14, 16, 18).

14. Produit de programme informatique comprenant des instructions qui, lorsque le programme est exécuté par un ordinateur, amènent l'ordinateur à exécuter les étapes selon l'une quelconque des revendications 8-13.
